(19) Europäisches Patentamt
European Patent Office
Office européen des brevets

(11) EP 2 669 008 A2

(12) EUROPEAN PATENT APPLICATION
published in accordance with Art. 153(4) EPC

(43) Date of publication:
04.12.2013 Bulletin 2013/49

(21) Application number: 12738891.6

(22) Date of filing: 26.01.2012

(51) Int Cl.:
B01J 27/199 (2006.01)
B01J 23/88 (2006.01)
B01J 27/19 (2006.01)
C07C 45/85 (2006.01)
C07C 47/22 (2006.01)

(86) International application number:
PCT/JP2012/052327

(87) International publication number:
WO 2012/102411 (02.08.2012 Gazette 2012/31)

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR

(30) Priority: 28.01.2011 JP 2011016304

(71) Applicant: NIPPON KAYAKU KABUSHIKI KAISHA
Tokyo 102-8172 (JP)

(72) Inventors:
- OKUMURA, Kimito
SanyoOnoda-shi, Yamaguchi 757-8686 (JP)
- KAWAGUCHI, Toru
SanyoOnoda-shi, Yamaguchi 757-8686 (JP)
- KOBAYASHI, Yasushi
SanyoOnoda-shi, Yamaguchi 757-8686 (JP)

(74) Representative: Gille Hrabal
Patentanwälte
Brucknerstrasse 20
40593 Düsseldorf (DE)

# (54) CATALYST FOR SELECTIVELY REDUCING SATURATED ALDEHYDE, AND PRODUCTION METHOD THEREFOR

(57) [Problem] Catalyst for use in selective reduction of propionaldehyde in acrolein and/or acrylic acid and/or acrylonitrile containing propionaldehyde and/or propionic acid and/or propionitrile at low concentration. In particular, a novel catalyst for selectively reducing propionaldehyde from acrolein containing the propionaldehyde.

[Solution] Catalyst for use in selective reduction of propionaldehyde in acrolein containing the propionaldehyde, characterized in that the catalyst contains Mo as an indispensable component, and at least one element selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al.

EP 2 669 008 A2

## Description

### Technical Field

This invention relates to a novel catalyst for selectively reducing saturated aldehyde, in particularly propionaldehyde (or propanal), and a process for preparing the catalyst.

In particularly, this invention relates to a novel catalyst for selectively reducing propionaldehyde from an acrolein-rich mixture (a mixture containing acrolein as a main component and secondary products including propionaldehyde as well as other compounds such as acrylic acid, acetaldehyde, acetone, phenols which are produced by oxidation of propylene and by dehydration of glycerin), when acrolein and/or acrylic acid and/or acrylonitrile containing low-concentration of propionaldehyde and/or propionic acid and/or propionitrile, is produced in gas phase or liquid phase, and a process for preparing the catalyst.

This invention relates also to a novel catalyst for selectively reducing propionaldehyde from acrolein which contains the propionaldehyde, and a process for preparing the catalyst.

In particularly, this invention relates to a novel catalyst for selectively reducing propionaldehyde from an acrolein-rich mixture obtained by dehydration reaction of glycerin, and a process for preparing the catalyst.

### Background Art

Acrolein is a key intermediate for the synthesis of methylmercapto-propionaldehyde and of methionine, a synthetic amino acid used as an animal feed supplement, which has emerged as a substitute for fishmeal. Acrolein is also a non-isolated synthetic intermediate of acrylic acid and acrylonitrile for which the importance of their applications and its derivatives is known. Acrolein also leads, via reaction with methyl vinyl ether then hydrolysis, to glutaraldehyde, which has many uses in leather tanning, as a biocide in oil well drilling and during the treatment of cutting oils, and as a chemical sterilant and disinfectant for hospital equipment. Acrolein also leads to pyridine or glutaraldehyde.

Acrylic acid is a material used in a variety of industrial products and is an important monomer and comonomer of industrial polymers such as polyacrylates and polyacrylamide produced by polymerization of acrylic acid and its derivative. One of the important applications of acrylic acid is high water absorption resins prepared by partial neutralization of a mixture of acrylic acid and sodium acrylate or other cation. In practice, acrylic acid is polymerized and the resulting polyacrylic acid is partly neutralized. These polymers or copolymer are utilized widely in various fields such as sanitation, detergent, coating material, varnish, adhesive, paper, fabric and leather.

Acrolein and acrylic acid are produced in industrial scale by a process for oxidizing propylene by using a catalyst in the presence of oxygen. Generally, this reaction is carried in gas phase. Acrylic acid is usually produced by two step reactions. In the first step, acrolein-rich product is prepared from propylene but little acrylic acid is produced in this stage. Acrylic acid is obtained by selective oxidation of acrolein in the second step.

The two step reaction is carried out in multi-tubular reactors under two different reaction conditions and catalysts each suitable for each reactor. There is no necessity to effect purification of acrolein obtained in the first step.

Starting material used in production of acrolein and acrylic acid is derives from petroleum and natural gas which are no regenerable fossil resources. However, it is very important to produce them from renewable sources to reduce the global warming gas. Such change is the responsibility of industry major powers and can contribute to relaxation of the environmental loads and reduction of global warming gas.

Glycerol is derived from plant oils in the production of biodiesel fuels or oleochemicals such as fatty acids or fatty alcohol or fatty esters. Glycerol is one of the raw materials envisaged as a substitute for propylene, glycerol being able to be subjected to a catalytic dehydration reaction in order to produce acrolein. Such a process makes it possible to thus respond to the concept of green chemistry within a more general context of protecting the environment. There are also many possible ways to access to renewable glycerol, for example by fermentation of sugars or by hydrogenolysis reactions.

The above process rout is very similar to the propylene oxidation process, because acrolein is prepared in the first step and the second step is carried out in the same reaction condition as the first step.

However, actual first step is different from the usual propylene oxidation process. In fact, a different solid catalyst from the propylene oxidation is used in the dehydration reaction in gas phase and in this process, much water together with acrolein-rich gas is supplied to the second step for producing acrylic acid. Still more, a composition of by-products also is very different due to completely different reaction mechanism.

Numerous catalyst systems have already been the subject of studies for the dehydration reaction of glycerol to acrolein.

Patent Document 1 (US 5,387,720) describes a process for producing acrolein by dehydration of glycerol, in liquid phase or in gas phase over acidic solid catalysts that are defined by their Hammett acidity The catalysts must have Hammett acidity below +2 and preferably below -3. These catalysts correspond, for example, to natural or synthetic siliceous materials, such as mordenite, montmorillonite and acidic zeolites; supports, such as oxides or siliceous materials, for example alumina ($Al_2O_3$), titanium oxide ($TiO_2$) covered by monobasic, dibasic or tribasic inorganic acids; oxides or mixed oxides such as gamma-alumina, $ZnO/Al_2O_3$ mixed oxide, or else heteropolyacids. The use of these catalysts would make it possible to solve the problem of formation of secondary products generated with the iron phosphate type catalysts described in Patent Document 12 (FR 695,931).

Patent Document 2 (WO2006/087084) shows that strong solid acid having Hammett index from -9 to -18 permits to produce acrolein at high activity by glycerin dehydration and to suppress degradation of catalyst.

Patent Document 3 (WO2009/044081) discloses a glycerin dehydration reaction effected in the presence of a catalyst containing oxygen, iron, phosphorus and alkali metal and more than one alkali-earth metals selected from a group comprising Al, Si, B, Co, Cr, Ni, V, Zn, Zr, Sn, Sb, Ag, Cu, Nb, Mo, Y, Mn, Pt, Rh and rare earth.

Patent Document 4 (WO2009/128555) discloses a glycerin dehydration reaction effected in the presence of a catalyst consisting of a compound in which protons in heteropolyacid are exchanged with more than one cation of elements selected from a group comprising elements belonging to Group 1 to Group 16 of periodic table.

Patent Document 5 (WO2010/046227) discloses a glycerin dehydration reaction effected in the presence of a catalyst containing oxygen, phosphorus and at least one element selected from a group comprising vanadium, boron and aluminum.

The catalysts proposed in these documents, however, have such problems as deposition of cokes and development of secondary reactions which generate hydroxy acetone, propionaldehyde, acetaldehyde, acetone, addition reaction of acrolein to the glycerin, polycondensation of glycerin, formation of cyclic glycerin ether, phenol, and poly aromatic compounds, which degrade the catalyst.

The presence of such by-products in acrolein, especially propionaldehyde is a serious problem in purification and separation stage. In fact, a considerable cost is required to obtain acrolein of high concentration. Still more, when acrolein containing propionaldehyde is used for producing acrylic acid, the propionaldehyde is oxidized to propionic acid which is difficult to be separated from acrylic acid. Namely, boiling points are 49°C of propionaldehyde and 141 °C of propionic acid and are very near to boiling points of acrolein of 53°C and acrylic acid of 141°C. Same problem exists when acrolein is used for producing methionine or acetal or acrylonitrile, since boiling points are 77°C of acrylonitrile and 97°C ofpropionitrile.

This problem becomes serious in the two processes for producing acrolein/acrylic acid or acrylonitrile from propylene or glycerin. It is true that propionaldehyde is formed in the glycerin dehydration reaction and in the propylene oxidation as by-products. However, in case of the glycerin dehydration, a larger amount of propionaldehyde is produced, comparing to the propylene oxidation, so that the selectivity is lowered.

Presence of a large amount of impurities narrow the final uses or applications of acrolein and acrylic acid produced. In particular, such impurity as not-polymerized saturated compounds changes the physical property of end products, will be causes of toxicity and corrosion, and result in increase of organic pollutants in acrylic resin and in the final products.

Therefore, there is a need to produce acrylic acid which satisfies the above requirements, or acrylic acid produced not from fossil fuel but from natural carbon sources, or such acrylic acid of high-quality that can be uses in production of a variety of synthetic polymers without complicated costly purification.

Many techniques are proposed to satisfy the above requirements. For example, Patent Document 3 (WO2009/044081) uses two catalysis layers in a system, a mixed glycerin gas is fed to the first catalyst layer to effect dehydration of glycerin at least partially with generation of by-products such as propionaldehyde. The resulting reaction gas mixture is passed through a second catalytic layer of iron phosphate to dehydrate unreacted glycerin and to convert propionaldehyde to acrolein. This method permits to reduce the content of propionaldehyde and to obtain acrylic acid of high-pure from acrolein, so that applications can be expanded. However, it is confirmed that reaction tubes are stuffed immediately when this catalyst is used.

Patent Document 5 (WO2010/046227) discloses use of two layered catalyst based on composite oxide of phosphorus and vanadium to prevent generation of propionaldehyde in acrolein. This catalyst, however, lacks the activity in usual reaction temperatures.

Patent Document 6 (WO2010/074177) discloses a process for producing acrylic acid from a mixture containing acrolein and propionaldehyde by using a solid catalyst containing Mo and V as indispensable components in gas phase. In this process, acrolein is converted into acrylic acid in gas phase reaction and propionaldehyde is converted into propionic acid and acrylic acid. In this acrolein oxidation catalyst, propionaldehyde is converted at the same speed as acrolein, so that propionaldehyde is converted mainly into propionic acid. However, the conversion from propionaldehyde to acrylic acid is such very low as 3%. The resulting acrylic acid contains many propionic acid, and hence it is necessary to carry out purification by recrystallization to remove propionic acid selectively.

A method to reduce propionic acid from acrylic acid is also proposed. For example, it is known a method for reducing propionic acid from a mixed gas containing acrylic acid obtained gas phase oxidation of propane or propylene in the presence of a composite metal oxide including at least one element chosen from Mo and/or Bi (Patent Document 7) or in the presence of a composite metal oxide for propionic acid removal including at least one element chosen from Mo and W (Patent Document 8). In these processes, high reaction temperature ( 300 to 500°C. in Patent Document 7) is required or a considerable quantity of acrylic acid is lost (over 6% in Patent Document 8).

It is also known a process for catalytically oxidize-dehydrating saturated aldehyde to unsaturated aldehyde.

In Non-patent Document 1 (Hargis et al, in I&EC product research and development, Vol 5, No.1, March1966. pp 72-75) , It is proposed to use arsenic, antimony or bismuthal oxide to convert saturated aldehyde to unsaturated aldehyde. In this process, propionaldehyde is converted to acrolein by using $Sb_2O_4$ as oxidizing agent. But, the conversion is 5% and the selectivity is merely 62%.

Patent Document 9 (US 4,381,411) discloses a process to use iron phosphate including at least one catalyst aid in oxidative dehydrogenation of saturated aldehyde to unsaturated aldehyde, or production of metacrolein from isobutyl aldehyde. Metacrolein is obtained at a yield of 52 to 80% and a conversion of 100%.

In Non-patent Document 2 (Kinetics and Catalysis, Vol44, No.2, 2003, pp.198-201), isobutyl aldehyde is obtained at the conversion of 80% at a selectivity of metacrolein of 82% by using iron phosphate catalyst. Ii is also reported that the selectivity is lower for Fe-P alone but the oxidation activity is increased when a minimum quantity of Mo is added to Fe-P at without spoiling the selectivity. However, there is no example to show this effect in oxidative dehydrogenation of propionaldehyde to acrolein. The selectivity will lower for Mo supported catalyst, when an amount of Mo is increased higher than 4% in catalyst.

Patent Document 10 (JP-54-046705) discloses a process for producing acrolein or unsaturated aldehyde like metacrolein and acrylic acid or unsaturated carboxylic acid like methacrylic acid, by oxidizing C3 and C4 like isobutyl aldehyde and propionaldehyde in gas phase in the presence of a catalyst containing Mo, P oxide and at least one element selected from Zn, Cu and Ag, and having a specific surface of at least 10 $m^2/g$ and supported on carner. In case of propionaldehyde, the conversion of propionaldehyde is 72.6%, the yield of acrolein is 40.6%, and the yield of acrylic acid is 12.5%. This indicated that it is difficult to convert propionaldehyde at high conversion with high selectivity of acrolein.

In Non-Patent Document 3 (Journal of Catalysis 195, 360-375, 2000), Ji Hu et al. reports oxidative dehydrogenation reaction of isobutyl aldehyde to methacrolein in PMo catalyst. In Figs. 1 and 2 which show the effect of temperature to reaction, it is shown that the conversion of isobutyl aldehyde can not be increased over 95%, even if a high reaction temperature is used. This indicated that it is difficult to more than 95 % of isobutyl aldehyde without affecting the selectivity of methacrolein.

In Non-Patent Document 4 (React. Kinet. Catal. Lett. Vol. 81, No. 2, 383-391, 2004), Cicmanec et al. repeated the same reaction by using CsPMo catalyst. Figs. 3 and 4 show the effect of the contact time to the conversion of isobutyl aldehyde and the yield of methacrolein and reveal that 90% is limitative of the conversion even at higher temperature range.

It is clear from above Patent Documents and non-Patent Documents that the conversion of saturated aldehyde to unsaturated aldehyde is yet at low level due to several problems including degradation of catalyst.

In Patent Document 11 (WO2009/127889), the yield of acrolein of 16.3% is obtained by using Mo-containing heteropolyacid at the conversion of glycerin of 91.3%. This means that Mo-containing heteropolyacid is not desirable in the production of acrolein from the glycerin. Inventor had no intention to use the catalyst to remove propionaldehyde as an impurity in acrolein.

[Prior arts]

[Patent Document]

[Patent Document 1] US 5,387,720
[Patent Document 2] WO2006/087084
[Patent Document 3] WO2009/044081
[Patent Document 4] WO2009/128555
[Patent Document 5] WO2010/046227
[Patent Document 6] WO2010/074177
[Patent Document 7] JP-A1-10-218831
[Patent Document 8] EP 2039674
[Patent Document 9] US 4381411
[Patent Document 10] JP-A1-54-046705
[Patent Document 11] WO2009/127889
[Patent Document 12] FR 695931

[Non-patent Document]

[Non-patent Document 1] Hargis et al, in I & EC product research and development, Vol. 5, No.1, March1966, pp 72-75
[Non-patent Document 2] Kinetics and Catalysis, Vol44, No.2,(2003) pp198-201
[Non-patent Document 3] Journal of Catalysis 195, 360-375(2000)
[Non-patent Document 4] React. Kinet. Catal. Lett. Vol. 81, No.2, 383-391(2004)

## Disclosure of Invention

### Technical Problems

Therefore, there is a need to develop such a catalyst that can reduce selectively propionaldehyde as an impurity from acrolein-rich mixture without big adverse effect to acrolein production. An object of this invention is to respond to this need.

Present inventors found that propionaldehyde can be selectively reduced from acrolein-rich mixture at high selectivity, by using a catalyst containing at least Mo as an indispensable element. Therefore, another object of this invention is to provide a catalyst that can produce acrolein and/or acrylic acid whose contents of propionaldehyde or propionic acid care very low.

Further object of this invention is to provide a catalyst for selectively reducing propionaldehyde when acrylic acid is produced from natural carbon resources or to solve the above problems of known catalyst used in glycerin dehydration to produce acrolein and acrylic acid from the glycerin by reducing the contents of propionaldehyde.

Still further object of this invention is to provide a novel catalyst that can selectively reduced the contents of propionaldehyde from acrolein-rich mixture, when acrolein and/or acrylic acid and/or acrylonitrile (containing propionaldehyde and/or propionic acid and/or propionitrile at low concentrations) are produced in gas phase or in liquid phase.

In particular, this invention provides a novel catalyst that can selectively reduced the contents of propionaldehyde in acrolein containing the propionaldehyde.

In particular, this invention provides a novel catalyst that can selectively reduced the contents of propionaldehyde acrolein-rich mixture produced by glycerin dehydration reaction.

Further object of this invention is to provide a process for producing the above catalyst.

### Technical Solution

Inventors tried to solve the above problems and studied with experiments, and finally found that propionaldehyde can be selectively reduced by passing an acrolein containing propionaldehyde through a catalyst containing Mo as an essential component, and containing at least one element selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al, preferably from a group comprising P, Si, W, Ti, Cr, Mn, Fe, Co, Ni, Zn, Sn, Bi, Sb, Ce, Mg, Cs and K in the presence of oxygen, and completed the present invention.

This invention has following features of (1) to (10) alone or in combination:

(1) A catalyst for selectively reducing the propionaldehyde from acrolein containing the propionaldehyde, containing Mo as an essential component, and at least one element selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al, preferably from a group comprising P, Si, W, Ti, Cr, Mn, Fe, Co, Ni, Zn, Sn, Bi, Sb, Ce, Mg, Cs and K.

(2) The catalyst comprises a compound represented by the following formula (1):

$$Aa\ Xb\ Yc\ Zd\ Oe \qquad (1)$$

in which A is at least one cation of elements selected from a group belonging to Group 1 to Group 16 of the periodic table, X is P or Si, Y is Mo, Z is at least one element selected from a group comprising Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K and Al, a, b, c and d satisfying respective ranges of $0 \leq a < 9$, $0 \leq b \leq 1$. $0 < c \leq 20$ and $0 < d \leq 20$, and e is a number determined by oxidation numbers of other elements.

(3) The catalyst comprises a compound represented by the following formula (1):

$$Mo_{12}\ X_b\ O_c \qquad (2).$$

in which X is at least one cation of elements selected from W, Ti, Cr, Mn, Fe, Co, Ni, Zn, Tl, Sn, Bi, Sb, Mg, K, Rb and Cs, b is a number of atoms of X and satisfies a range of $0 < b < 30$, and c is a number determined by oxidation numbers of other elements.

(4) The compound comprises mainly heteropolyacid containing Mo as an indispensible component.

(5) The compound comprises mainly heteropolyacid containing Mo as an indispensible component and protons in the heteropolyacid are exchanged with at least one cation of elements selected from a group belonging to Group 1 to Group 16 of the periodic table.

(6) The cation is at least one alkali metal cation.

(7) The alkali metal is cesium.

(8) At least one salt of elements selected from a group belonging to Group 1 to Group 16 of the periodic table is added in addition to the compound for use.

(9) The compound is supported on carrier for used.

(10) The catalyst is produced by firing or calcination.

In the context of this specification, "propionaldehyde is selectively reduced" is understood that propionaldehyde is converted to higher than acrolein on a catalytic layer of this invention (propionaldehyde conversion/acrolein conversion >1) or more than 50 % of propionaldehyde is not converted to propionic acid.

In the context of this specification, "acrolein-rich mixture" means those product by known processes disclosed in the above-mentioned patent documents non-patent documents.

## Advantageous Effect

When the catalyst according to this invention is used, it is possible to reduce selectively propionaldehyde from acrolein-rich mixture which is obtained by glycerin dehydration and/or propylene oxidation, and to reduce the content of propionaldehyde in acrolein, acrylic acid, acrylate and these polymer products, so that products of high-quality can be obtained. And, it is possible to omit complicated purification and separation stages for eliminate propionaldehyde from the acrolein-rich mixture, so that this invention has a very important industrial value from a viewpoint of simplification of purification stage.

## Mode for Carrying out the Invention

The catalyst for reducing propionaldehyde selectively from acrolein containing the propionaldehyde according to this invention comprises Mo as an indispensable component and at least one element selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al, preferably fro a group comprising P, Si, W, Ti, Cr, Mn, Fe, Co, Ni, Zn, Sn, Bi, Sb, Ce, Mg, Cs and K.

The catalyst for reducing propionaldehyde selectively from acrolein containing the propionaldehyde according to this invention may be Mo-containing composite metal oxide having following composition (1):

$$Aa\ Xb\ Yc\ Zd\ Oe \qquad (1)$$

in which,

A is at least one cation selected from elements belonging to the Group I to Group 16 in the Periodic Table,
X is P or Si,
Y is Mo,
Z is at least one element selected from a group comprising W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al, preferably fro a group comprising P, Si, W, Ti, Cr, Mn, Fe, Co, Ni, Zn, Sn, Bi, Sb, Ce, Mg, Cs and K,
"a", "b", "c" and "d" in the formula each satisfy respective range of $0 \leq a < 9$, $0 \leq b \leq 1$, $0 < c \leq 20$ and $0 < d \leq 20$, and "d" is a value determined by oxidation numbers of other elements.

This catalyst includes Mo-containing composite metal oxides, Mo-containing heteropolyacids and their salts and their supported catalysts.

The Mo-containing composite metal oxide of this invention comprises a compound represented by the general formula (2):

$$Mo_{12}X_bO_c \qquad (2)$$

in which,

X is at least one element selected from a group comprising W, Ti, Cr, Mn, Fe, Co, Ni, Tl, Zn, Sn, Bi, Sb, Mg, K, Rb and Cs,
"b" is a number of atom of X and satisfies a range of $0 < b < 30$, and
"c" is a value determined by oxidation numbers of other elements.

A desirable catalyst is a catalyst having a catalytic component defined in claim 1 of Japanese Patent No. 3,793,317 (Patent Document 13).

[Patent Document 13] Japanese Patent No. 3,793,317

Namely, the catalytic component has following composition (3):

$$Mo_a\ Bi_b\ Ni_c\ Co_d\ Fe_f Yg\ Z_h\ O_x \qquad (3)$$

in which,

Y is at least one element selected from a group comprising Y Sn, Zn, W, Cr, Mn, Mg, Sb and Ti,
Z is at least one element selected from a group comprising K, Rb, Tl and Cs, and
a, b, c, d, e, f, g, h and x are each respectively number of atoms of Mo, Bi, Ni, Co, Fe, Y, Z and oxygen and satisfies respective range of a = 12, b = 0.1 to 7, c + d = 0.5 to 20, f = 0.5 to 8, g = 0 to 2 and h = 0.01 to 0.5, and x is a value determined by oxidation numbers of other elements.

By using this catalyst of this invention which can reduce propionaldehyde selectively, propionaldehyde can be converted at higher activity and higher selectivity from acrolein containing the propionaldehyde.
The catalyst comprising the Mo-containing heteropolyacids or their salts for reducing propionaldehyde selectively from acrolein containing the propionaldehyde according to this invention can be heteropolyacid containing Mo as an indispensable component, or those containing Mo as an indispensable element and in which protons of heteropolyacid are exchanged with at least one cation of metals selected from elements belonging to Group 1 to Group 16 of the periodic table and having following composition (4):

$$Aa\ Xb\ Yc\ Zd\ Oe \qquad (4)$$

in which

A is cation of metals selected from elements belonging to Group 1 to Group 16 of the periodic table,
X is X is P or Si,
Y is Mo,
Z is at least one element selected from a group comprising W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al, preferably from a group comprising W, Ti, Cr, Mn, Fe, Co, Ni, Zn, Sn, Bi, Sb, Ce, Mg, Cs and K,
$0 \leq a < 9$, $b = 1$ , $0 < c \leq 20$ and $0 < d \leq 20$ and e is a value determined by oxidation numbers of other elements.

The heteropolyacid is known as having several structures such as Keggin type, Dawson type and Anderson type and has generally high molecular weight of from 700 to 8,500. Term of the heteropolyacid covers their dimer complex also. Salts of heteropolyacid are understood as metal salts of heteropolyacid and onium salts which are produced by condensation of more than two inorganic oxoacids.
The acidic metal salt of heteropolyacid may be salts of metals belonging to Group 1 to Group 16 of the periodic table such as salts of sodium, potassium, rubidium, cesium, magnesium, calcium, strontium, barium, scandium, yttrium, lanthanide, titanium, zirconium, hafnium, chromium, manganese, rhenium, iron, ruthenium, osmium, cobalt, nickel, palladium, platinum, copper, silver, gold, zinc, gallium, thallium, germanium, tin, leads, bismuth and tellurium. The acidic onium salt of heteropolyacid may be amine salt, ammonium salt, phosphonium salt, sulfonium salt or the like.
The catalyst used in selective reduction of propionaldehyde in acrolein containing the propionaldehyde according to this invention is characterized in that the heteropolyacid or its salt contains Mo as an indispensable component.
Ion of molybdenum becomes oxo acid in water and the oxo acid polymerize to form polyoxo acid of high polymer. In this case, not only same or similar oxo acids polymerize each other but also different oxo acids may be polymerized around the above oxo acids to form a so-called heteropolyacid having a polynuclear structure in which more than two types of oxo acids are coagulated. An atom for a center oxo acid is called as "hetero atom", while atoms which are polymerize around the central oxo acid are called as "poly atom". The hetero atom may be silicon, phosphorus, arsenic, sulfur, iron, cobalt, boron, aluminum, germanium, titanium, zirconium, cerium and chromium. Among them, phosphorus or silicon is preferable. The poly atom may be molybdenum, tungsten, vanadium, niobium and tantalum. Among them, molybdenum is an essential component and tungsten and vanadium are preferable.

The heteropolyacid containing Mo as an indispensable element used in selective removal of propionaldehyde in acrolein containing the propionaldehyde according to this invention, or heteropolyacid in heteropolyacid salt is preferred phosphomolybdic acid and silocomolybdic acid. Other preferable type is those having phosphorus or silicon as hetero atom and having molybdenum as the poly atom, so that molybdenum and tungsten or molybdenum and vanadium form respectively a mixed configuration. It can be a mixed configuration consisting of a plurality of different types.

The catalyst for reducing propionaldehyde selectively from acrolein containing propionaldehyde according to this invention may be consisting mainly of alkali metal salt of heteropolyacid in which at least part of its proton is exchanged with alkali metal cation.

In the catalyst for reducing propionaldehyde selectively from acrolein containing propionaldehyde according to this invention, it is preferable to use cesium as the alkali metal and to exchange at least part of protons in the heteropolyacid with cesium cation.

Another preferred catalyst for reducing propionaldehyde selectively according to this invention consists mainly of alkali metal salt of heteropolyacid in which at least part of its protons is exchanged with cesium cation and a part of remaining protons is exchanged with cation of metals belonging to Group 1 to Group 16 of the periodic table.

The catalyst that can eliminate propionaldehyde selectively according to this invention can be used to reduce the content of propionaldehyde at high activity and high selectivity in acrolein which contains the propionaldehyde. When at least a part of the protons of heteropolyacid is exchanged with cesium cation, the resistance to water is improved in comparison with non-exchanged water soluble heteropolyacid, so that the life of catalyst and the recovery rate of catalyst are improved.

An amount of an aqueous solution of inorganic salt used in the above cation exchange is determined in such a manner that an electric charge of cation added becomes equal to an electric charge of heteropolyanion. For example, when heteropolyanion have a valence of three and a cation to be added has a valence of one, an amount of less than three equivalents of the cation is added. When a cation to be added has a valence of three, an amount of the cation is determined is added to the heteropolyanion. When a plurality of cations is added, an amount of the cation is determined in such a manner that the total of electric charge of the cations becomes equal to an electric charge of heteropolyanion. If an amount of aqueous solution of inorganic salts for cation exchange becomes excess, the activity of catalyst will be spoiled and the life of catalyst will be shorten.

The catalyst that can eliminate propionaldehyde selectively according to this invention can contain further at least one compound of element selected from a group comprising Group 1 to Group 16 of the periodic table, in addition to the above fact that at least part of its protons is exchanged with cesium cation of metals belonging to Group 1 to Group 16 of the periodic table.

The above compound of element selected from a group comprising Group 1 to Group 16 of the periodic table may be metal salt and onium salt. Examples of the metal salt are salts of tellurium, platinum, palladium, iron, zirconium, copper, cerium, silver and aluminum. Examples of the onium salt are amine salt, ammonium salt, phosphonium salt and sulfonium salt. Materials for the metal salts or onium salts may be nitrate, carbonate, sulfate, acetate, oxides, halide and hydroxide of the metals or onium, but are not limited to them. An amount of the metal salt or salt or metal or onium salt with respect to heteropolyacid is from 0.01 % by weight to 60 % by weight, preferably from 0.01 % by weight to 30 % by weight.

The catalyst for selectively reducing propionaldehyde from acrolein containing propionaldehyde according to this invention may be supported on carrier. Namely, the above mentioned Mo-containing metal oxide or Mo-containing composite heteropolyacid or their salts can be supported on the carrier (supported catalyst of this invention).

The carrier may be made of silica, diatomaceous earth, alumina, silica alumina, silica magnesia, zirconia, titania, magnesia, zeolite, silicon carbide and carbide. The carrier can be made of one of these carriers or mixtures of these carriers or their complex. Active substance can be used effectively by using such carrier. An amount of support is from 0 % by weight to 90 % by weight, preferably 5 % by weight to 90 % by weight to the total of Mo-containing composite metal oxide or Mo-containing heteropolyacid or their salts and of the carrier.

The catalyst can have any shape such as granule and powder. In case of for gas phase reaction, it can be molded into a form of sphere, cylinder, hollow cylinder or bars, by using a molding aid if necessity. It is also possible to shape the catalyst together with carrier and optional molding aid. Example of a size of molded catalyst is 1 to 10 mm for a fixed bed catalyst and a particle size of less than 1 mm for a fluidized bed catalyst.

Mo-containing composite metal oxide catalyst according to this invention can be prepared by any known technique such as coprecipitation method by using such a material as nitrate, ammonium salt, hydroxides and oxide of metal elements which constitute the catalytic component.

The Mo-containing heteropolyacid or their salts also can be prepared by any known technique. In practice, an aqueous solution of Mo-containing heteropolyacid is prepared. Or, adsorptive water of Mo-containing heteropolyacid and crystal water can be eliminated partially or perfectly under vacuum or drying before the aqueous solution of Mo-containing heteropolyacid is prepared. The resulting aqueous solution of Mo-containing heteropolyacid is added with an aqueous solution of halide, carbonate, acetate, nitrate, oxalate, phosphate, sulfate, hydroxide of metal of other element or of onium and then is subjected to evaporation to dryness or filtration or drying under reduced pressure to obtain a solid. This solid is then fired or calcinated to obtain a catalyst that can reduce propionaldehyde selectively.

The catalyst comprising Mo-containing heteropolyacid or its salt for selective reducing the content of propionaldehyde can be anhydride or hydrate. That is to say, it can be used without pretreatment or after pretreatment such as firing or calcination and drying under reduced pressure, before it is used for the selective removal reaction of propionaldehyde. The firing can be carried out under an atmosphere of air or inert gas such as nitrogen, helium and argon or in a mixed gas of such inert gas and oxygen. The firing can be done in any furnace such as muffle furnace, rotary kiln and fluidized bed furnace. Or, the firing can be done in a reaction tube used for selective reduction reaction of propionaldehyde. The firing temperature is usual 150 to 900°C, preferably 200°C to 700°C. and more preferably 200°C to 600°C for a firing time of 0.5 to 10 hours.

Acrolein-rich mixture used in this invention is understood as a mixture which is produced by catalytic oxidation of propylene (or, of other no fossil resources) or dehydration reaction of glycerin in gas phase or liquid phase, preferably in gas phase or dehydration reaction of hydroxy propionaldehyde.

Reaction conditions of the glycerin dehydration reaction to acrolein are described in Patent Document 14 (WO2006/087083), Patent Document 1 (WO2006/087084), Patent Document 3 (WO2009/128555) or Patent Document 4 (WO2010/046227). In particular, it is preferable to use a catalyst as a glycerin dehydration catalyst having Hammett index of lower than +2.

[Patent Document 14] WO2006/087083

Reaction conditions of the propylene oxidation to acrolein are scribed in non-Patent Document 5 (SRI, Process Economic Program no6D). Preferred reaction conditions are those described in Patent Document of 13 (Japanese Patent No. 3,793,317).

[Non-Patent Document 5] SRI, Process Economic Program no 6D

Namely, a mixed gas comprising 1 to 10 volume %, preferably 4 to 9 volume % of propylene, 3 to 20 volume %, preferably 4 to 18 volume % of molecular oxygen, 0 to 60 volume %, preferably 4 to 50 volume % of vapor, and 20 to 80 volume %, preferably 30 to 60 volume % of inert gas (nitrogen, carbon dioxide etc) is fed to a reaction tube filled with a catalyst for propylene oxidation to acrolein at a temperature of 250 to 450°C, under a pressure of ambient pressure to 10 atm at a space velocity of 300 to 5000$hr^{-1}$, preferably 800 to 2000$hr^{-1}$.

The content of propionaldehyde in the acrolein-rich mixture is generally less than 0.2 in term of a molar ratio of a mole quantity of propionaldehyde to a mole quantity of acrolein. In other words, the content of propionaldehyde in term of a mole ratio of propionaldehyde to acrolein is in a range of 1/1000 to 1/5, preferably in a rage of 1/2000 to 1/10.

The acrolein-rich mixture is understood in this invention, as a mixture containing, in addition to acrolein, by-products such as water, oxygen, propionaldehyde, propionic acid, hydroxy acetone, acetaldehyde and acetone, adduct product of glycerin acrolein conversion, copolymers of glycerin, ringed glycerin ether, hydroxy propionaldehyde, phenol, aromatic polymer, propane, propylene, acrylic acid, carbon monoxide, carbon dioxide, inert gas such as nitrogen, helium and argon. Physical properties of the by-products and compositions of the acrolein-rich mixture depend on material used in production of acrolein.

When propylene is used as a material for oxidation reaction to produce acrolein, the acrolein-rich mixture may contain propane. When glycerin is used as a material for the dehydration reaction, a large amount of water may be contained in acrolein. A proportion of water depends on a concentration of glycerin used.

A concentration of acrolein in the mixed gas to be passed through the catalyst according to this invention is 1 to 30 mol %, preferably 1 to 12 mol %, and more preferably 4 to 10 mol %.

The selective reduction of propionaldehyde according to this invention is effected under oxygen-containing gas such as oxygen or air. Usually, oxygen exists in the mixed gas. If the oxygen concentration is very low, oxygen can be added. The oxygen concentration is 1 to 10 mol %, preferably 3 to 7 mol %.

A reaction temperature for carrying out the selective reduction of propionaldehyde according to this invention is from 250°C. to 400°C and preferably from 280°C to 350°C. and a pressure is in absolute pressure from 1 bar to 5 bar, preferably from 1 bar to 2 bars.

The reactant gas is supplied at a rate of, in term of the space velocity GHSV, from 1000 to 40000$h^{-1}$, preferably

from 5000 to 20000$h^{-1}$. The selectivity will become lower if GHSV is lower than 1000$h^{-1}$ while the conversion will become lower if GHSV exceeds 40000$h^{-1}$.

The process for selective reduction of propionaldehyde according to this invention can be carried out in gas phase or liquid phase, preferably in gas phase. For the gas phase reaction, there are a variety of reactors such as fixed bed, fluidized bed, circulating fluidized bed and moving bed. Fixed bed or fluidized bed is desirable.

The catalyst can be filled in the above reactors as following for example:

(1) When production of an acrolein-rich mixture and the selective reduction of propionaldehyde are carried out in series in two reactors connected in tandem, each reactor is filled with a catalyst for acrolein production and a catalyst for the selective reduction of propionaldehyde, so that acrolein production and selective reduction of propionaldehyde are effected in each reactor separately.

(2) When production of an acrolein-rich mixture and the selective reduction of propionaldehyde are carried in a single type reactor, for a case in which gas circulates down-flow from an upper part to a lower part of a reaction tube, an upper layer is filled with the catalyst for acrolein production, while the a lower layer is filled with the catalyst for the selective reduction of propionaldehyde, so that production of acrolein and the selective reduction of propionaldehyde can be done in a single reactor.

The selective reduction of propionaldehyde is effected at a reaction temperature which is basically different from a temperature of acrolein production, so that it is desirable to pack each catalyst in a separate reactor having respective different temperature. In case of a single fixed bed, for a case in which gas circulates up-flow from a lower part to an upper part of a reaction tube, the catalyst for selective reduction of propionaldehyde is placed at an upper layer than a layer of the catalyst for the acrolein production. When a multi-tubular reactor for acrolein production is used, and gas circulates up-flow from a lower part to an upper part, the catalyst for selective reduction of propionaldehyde is placed an upper part of the multi-tubular reactor, namely at an upper part of the catalyst for the acrolein production.

The catalyst according to this invention permits to reduce the concentration of propionaldehyde in acrolein to lower than 5,000 ppm, preferably lower than 1,000 ppm. By using the catalyst for selection reduction of propionaldehyde according to this invention, it is possible to produce acrolein which contains propionaldehyde at a low concentration, and hence this invention relates also to produce acrylic acid containing propionic acid at a low concentration by oxidation reaction. In fact, it is possible to reduce a concentration propionic acid in acrylic acid to lower than 5,000 ppm, preferably lower than 1,000 ppm.

Generally, the oxidation reaction from acrolein to acrylic acid is effected in the presence of oxygen or a mixed gas with molecular oxygen, at a temperature of in a range of from 200°C to 350°C., preferably from 250°C. to 320°C, under a pressure of 1 bar to 5 bar absolute pressure, and in the presence of a catalyst containing at least one element selected from a group comprising Mo, V, W, Re, Cr, Mn, Fe, Co, Ni, Cu, Zn, Sn, Te, Sb, Bi, Pt, Pd, Ru and Rh which is in a form of metal, oxide, sulfate or phosphate. This catalyst for oxidation reaction can be prepared by compounding mainly Mo and/or V and/or W and/or Cu and/or Sb and/or Fe. This oxidation reaction catalyst may be supported on carrier such as zirconia, silica, alumina, titania, steatite and silicon carbide or their mixtures.

The catalyst for selective reduction of propionaldehyde according to this invention permits to produce acrolein containing propionaldehyde at a lower concentration, and hence this invention relates also to ammoxidation for producing acrylonitrile containing propionitrile at a low concentration. In fact, it is possible to reduce a concentration of propionitrile in acrylonitrile to lower than 5,000ppm, preferably lower than 1,000 ppm.

Now, this invention will be explained in more detail with referring Examples. However, this invention is not limited to following Examples as long as the aim is not exceeded. In the following Examples and Comparison Examples, % means mole %.

## Examples

### Preparation of catalyst

### Example 1:

### CsPMo

100g of phosphomolybdic acid was dissolved in 200ml of pure water and stirred at ambient temperature for 2 hours, while 32.7g of aqueous solution of 48.5 % by weight of CsOH was diluted in 20ml of pure water. The resulting aqueous solution of CsOH was added to the aqueous solution of phosphomolybdic acid drop-wise under stirring at ambient temperature for 2 hours. The resulting yellow slurry was dried by a rotary evaporator under reduced pressure at 60 °C to obtain a powder, which was then further dried by a drier under ambient pressure at 120°C for 10 hours. The resulting dried powder has following composition:

$$Cs_{2.5}P_{1.0}Mo_{12}.$$

This powder was further fired in Muffle furnace at 250 °C in air for 3 hours.

Example 2

CsPWMo

50g of phosphorus tungusto molybdic acid was dissolved in 20ml of pure water and stirred at ambient temperature for 2 hours, while 13.4g of aqueous solution of 48.5 % by weight of CsOH was diluted in 50ml of pure water. The resulting aqueous solution of CsOH was added to the aqueous solution of phosphomolybdic acid drop-wise under stirring at ambient temperature for 2 hours. The resulting yellow slurry was dried by a rotary evaporator under reduced pressure at 60 °C to obtain a powder, which was then further dried by a drier under ambient pressure at 120°C for 10 hours. The resulting dried powder has following composition:

$$Cs_{2.5}P_{1.0}W_6Mo_6.$$

This powder was further fired in Muffle furnace at 250°C in air for 3 hours.

Example 3

MoVPCuAs

100g of molybdenum trioxide, 6.3g of vanadium pentoxide, 1.1g of copper oxide, 8.0g of 85 % by weight orthophosphoric acid and 1.8g of 60 wt% arsenic acid were mixed in 1000ml of pure water. The resulting mixture was added with hydrogen peroxide and refluxed for 6 hours until a clear reddish brown solution was obtained. From the resulting solution, a small amount of insoluble material was removed and then the solution was dried up by evaporation by using a water bus. The resulting dried powder has following composition:

$$Mo_{10}V_{1.0}P_{1.0}Cu_{0.2}As_{0.2}.$$

This powder was further fired in Muffle furnace at 310 °C in air for 5 hours.

Example 4

MoVPCuAsSb

300g of molybdenum trioxide, 11.37g of vanadium pentoxide, 3.31g of copper oxide, 8.32g of copper acetate, 28.82g of 85 % by weight orthophosphoric acid and 24.64g of 60 wt% arsenic acid were mixed in 1900ml pure water. The resulting mixture was refluxed for 6 hours at a temperature from 95 °C at 100 °C until a clear reddish brown solution was obtained. Into the resulting solution, 1.52g of antimony trioxide was added and the solution was heated at a temperature from 95 °C at 100 °C for 3 hours. Then, the solution was dried up by evaporation by using a water bus. The resulting dried powder has following composition:

$$Mo_{10}V_{0.6}P_{1.2}Cu_{0.4}As_{0.5}Sb_{0.05}.$$

This powder was further fired in Muffle furnace at 310 °C in air for 5 hours.

Example 5

MoVPCuSbCs

200g of molybdenum trioxide, 8.84g of vanadium pentoxide and 17.61g of 85 % by weight orthophosphoric acid were added to 1200ml of pure water. The resulting mixture was refluxed for 5 hours at a temperature of from 95 °C at 100 °C until a clear reddish brown solution was obtained. Into the resulting solution, 6.07g of antimony trioxide and the solution was heated further at a temperature of from 90 °C to 100 °C for 2 hours to dissolve the antimony trioxide to obtain a solution of dark blue. The resulting solution was cooled to a temperature of from 15 °C to 20 °C. Then, into the resulting solution, a solution in which 13.33g cesium acetate was dissolved in 150ml pure water and a solution in which 16.06g antimony acetate was dissolved in 150ml pure water were added gradually under stirring. Into the resulting slurry, a solution in which 11.09g of cupric acetate monohydrate was dissolved in 170ml of pure water was added and the slurry was aged a temperature of from 15 °C to 20 °C until a solution of blue-green was obtained. Then, the resulting slurry was dried up by evaporation by using a water bus. The resulting dried powder has following composition:

$$Mo_{10}V_{0.7}P_{1.1}Cu_{0.4}Sb_{0.3}CS_{0.5}(NH_4)_{1.5}.$$

This powder was further fired in Muffle furnace at 310 °C in air for 5 hours.

Evaluation and Results

Example 6

Selective removal of propionaldehyde from acrolein

CsPMo prepared in Example 1 was compacted, crushed and passed through a sieve to obtain granules of 35 to 48 meshes. The resulting catalyst granules were packed in a 2.0ml of SUS reaction tube (13mm inner diameter) to form a fixed catalytic layer.

A mixed solution of acrolein and propionaldehyde was pre-heated in a vaporizer heated at 270 °C and was fed directly to the catalytic layer together with oxygen and nitrogen at ambient pressure. The reactor containing the catalyst was heated at 270 °C. The feed stream have following composition: acrolein : propionaldehyde : oxygen : nitrogen : water = 6.0 mol % : 0.06 mol % : 3.8 mol % : 14 mol % : 76 mol %. The total gas flow rate was 31 L/hr. GHSV was 15,000 $h^{-1}$. The catalytic layer ran for 5 hours without pressurization.

To determine a composition of products at an outlet of the reaction tube, the products were collected in a condenser as condensates and gaseous products were also collected. The quantitative determination of all components was carried out by a gas chromatograph (HP6890 Agilent, FFAP column, FID detector, CP4900 Varian, Silica plot and Molecular Sieve 5 A, TCD detectors). Each product was corrected by the gas chromatograph to obtain absolute contents of products for determining the composition of the products.

The conversion (acrolein or propionaldehyde) of material and the yield and the conversion of objective substances were calculated by following equations:

The conversion of material (%) = (a mole number of material reacted / a mole number of material fed) * 100

The yield of acrylic acid (%) = (a mole number of acrylic acid obtained / a mole number of acrolein fed) * 100

The yield of propionic acid (%) = (a mole number of propionic acid obtained / a mole number of propionaldehyde fed) * 100

Relative removal ratio of propionaldehyde = the conversion of propionaldehyde / the conversion of acrolein.

Results are summarized in [Table 1].

Example 7

The same operation as Example 6 was repeated in the fixed bed at ambient pressure for 5 hours, but GHSV was changed to 14000$h^{-1}$ and the vaporizer and electric furnace were heated at 300 °C. Result is shown in [Table 1].

Example 8

The same operation as Example 6 was repeated in the fixed bed at ambient pressure for 5 hours, but GHSV was changed to 4400$h^{-1}$ and the vaporizer and electric furnace were heated at 270 °C. Result is shown in [Table 1].

Example 9

The same operation as Example 6 was repeated in the fixed bed at ambient pressure for 5 hours, but GHSV was changed to 4400$h^{-1}$ and the vaporizer and electric furnace were heated at 300 °C. Result is shown in [Table 1].

Example 10

The same operation as Example 6 was repeated in the fixed bed at ambient pressure for 5 hours, but catalysts was changed to CsPWMo of Example 2, GHSV was changed to 5500$h^{-1}$ and the vaporizer and electric furnace were heated at 250 °C. Result is shown in [Table 1].

Example 11

The same operation as Example 6 was repeated in the fixed bed at ambient pressure for 5 hours, but catalysts was changed to MoVPCuAs of Example 3, GHSV was changed to 5000$h^{-1}$ and the vaporizer and electric furnace were heated at 350 °C. Result is shown in [Table 1].

Table 1

| Example | 6 | 7 | 8 | 9 | 10 | 11 |
|---|---|---|---|---|---|---|
| Catalyst | CsPMo | CsPMo | CsPMo | CsPMo | CsPWMo | MoVPCuAs |
| GHSV $h^{-1}$ | 15,000 | 14,000 | 4,400 | 4,400 | 5,500 | 5,000 |
| Temperature of vaporiser and oven (°C) | 270 | 300 | 270 | 300 | 250 | 350 |
| Propionaldehide conversion (%) | 90 | 82 | 99 | 100 | 92 | 65 |
| Propionic acid yield (%) | 10 | 12 | 11 | 9 | 13 | 15 |
| Acrolein conversion (%) | 11 | 25 | 28 | 65 | 53 | <5 |
| Acrylic acid yield (%) | 7 | 22 | 13 | 64 | 36 | 5 |
| propionaldehyde elimination (relative ratio) | 7.9 | 3.2 | 3.5 | 1.5 | 1.7 | >13 |

Example 12

The same operation as Example 6 was repeated in the fixed bed at ambient pressure for 5 hours, but catalysts was changed to MoVPCuAs of Example 3, GHSV was changed to 4700$h^{-1}$ and the vaporizer and electric furnace were heated at 385 °C. Result is shown in [Table 2].

Example 13

The same operation as Example 6 was repeated in the fixed bed at ambient pressure for 5 hours, but catalysts was changed to MoVPCuAsSb of Example 4, GHSV was changed to 4400$h^{-1}$ and the vaporizer and electric furnace were heated at 345 °C. Result is shown in [Table 2].

Example 14

The same operation as Example 6 was repeated in the fixed bed at ambient pressure for 5 hours, but catalysts was changed to MoVPCuSbCs of Example 5, GHSV was changed to 5500$h^{-1}$ and the vaporizer and electric furnace were heated at 250 °C. Result is shown in [Table 2].

Table 2

| Example | 12 | 13 | 14 |
|---|---|---|---|
| Catalyst | MoVPCuAs | MoVPCuAsSb | MoVPCuSbCs |
| GHSV $h^{-1}$ | 4,700 | 4,400 | 5,500 |
| Vaporiser and oven temperature (°C) | 385 | 345 | 250 |
| Propionaldehyde conversion (%) | 97 | 14 | 98 |
| Propionic acid yield (%) | 14 | 14 | 13 |
| Acrolein conversion (%) | 23 | <5 | 29 |
| Acrylic acid yield (%) | 20 | 1 | 15 |
| Relative ratio of Propionaldehyde | 4.2 | >3 | 3.4 |

Example 15

The same operation as Example 6 was repeated in the fixed bed at ambient pressure for 5 hours, but catalysts was changed to FeMo catalyst (MFM3-MS) obtained from MAPCO, GHSV was changed to $10000h^{-1}$ and the vaporizer and electric furnace were heated at 305 °C. Result is shown in [Table 3].

Example 16

The same operation as Example 6 was repeated in the fixed bed at ambient pressure for 5 hours, but catalysts was changed to FeMo catalyst of Example 15, GHSV was changed to $20000h^{-1}$ and the vaporizer and electric furnace were heated at 330 °C. Result is shown in [Table 3].

Example 17

The same operation as Example 6 was repeated in the fixed bed at ambient pressure for 5 hours, but catalysts was changed to FeMo catalyst of Example 15, and the composition of the feed gas was changed to acrolein : propionaldehyde : oxygen : nitrogen : water = 6.0 mol % : 0.062 mol % : 6.7 mol % : 69 mol % : 18 mol %. Result is shown in [Table 3].

Example 18

The same operation as Example 6 was repeated in the fixed bed at ambient pressure for 5 hours, but catalysts was changed to MoBiNiFeCoK which is a catalyst for propylene oxidation (a product of NIPPON KAYAKU) prepared according to Example 1 of Japanese Patent No. 3,793,317 and GHSV was changed to $5,800h^{-1}$ and the vaporizer and electric furnace were heated at 325 °C. Result is shown in [Table 3].

Example 19 (Comparative Example)

The same operation as Example 6 was repeated in the fixed bed at ambient pressure for 5 hours, but catalysts was changed to $TiO_2$(ST31119) (a product of Saint-Gobain) and GHSV was changed to $3,700h^{-1}$ and the vaporizer and electric furnace were heated at 30 °C. Result is shown in [Table 3].

This catalyst of Example 19 (Comparative Example) shows higher the acrolein conversion than the propionaldehyde conversion, in comparison to the catalyst according to this invention, which reveal that selective elimination of propionaldehyde is poor.

Table 3

| Example | 15 | 16 | 17 | 18 | 19 (Comparative) | |
|---|---|---|---|---|---|---|
| Catalyst | FeMo | | | BiMoFe | $TiO_2$ | |
| GHSV $h^{-1}$ | 10,000 | 20,000 | 20,000 | 5,800 | 1,500 | 3,700 |
| Vaporiser and oven temperature (°C) | 305 | 330 | 330 | 325 | 270 | 300 |

(continued)

| Example | 15 | 16 | 17 | 18 | 19 (Comparative) | |
|---|---|---|---|---|---|---|
| Catalyst | FeMo | | | BiMoFe | $TiO_2$ | |
| Propionaldehyde conversion (%) | 98 | 99 | 99 | 96 | <5 | 16 |
| Propionic acid yield (%) | 3 | 6 | 6 | 8 | 0 | 8 |
| Acrolein conversion (%) | 9 | <6 | 11 | <5 | 5 | 30 |
| Acrylic acid yield (%) | 0.5 | 2 | 2 | 3 | 0 | 1 |
| Relative ratio of Propionaldehyde | 11 | >15 | 9 | >19 | | 0.6 |

Example 20 (Comparative Example)

The same operation as Example 6 was repeated, but catalysts was changed to FePSr which was prepared according to Example 3 of WO 2009/44081 and GHSV was changed to 4,000$h^{-1}$ and the vaporizer and electric furnace were heated at 280 °C. Reaction was stopped because an internal pressure of a reaction system rose sharply.

Example 21

Preparation of acrolein by glycerin dehydration reaction

A catalyst PW/$TiO_2$ for glycerin dehydration reaction was prepared as following: 89mg of 85 wt% phosphoric acid and 2.33g of meta ammonium tungstate were dissolved in 7.5g of pure water to obtain an aqueous solution of tungstophosphoric acid. 7.6g of the resulting aqueous solution of tungstophosphoric acid was sprayed over 15.4g of $TiO_2$ powder which was prepared by crushing Anatase $TiO_2$ pellet (ST31119, product of Saint-Gobain) to sizes of 35 to 48 meshes. The resulting powder was dried at 110°C for 2 hours, and fired under nitrogen atmosphere at 500°C for 3 hours to obtain a supported catalyst of 10wt% tungstophosphoric acid supported on $TiO_2$. A SUS reaction tube (inner diameter of 13mm) was filled with 2.4ml of CsPMo catalyst of Example 1 and then with 9.6ml of the above PW/$TiO_2$.

An aqueous solution of 50wt% glycerol was fed a vaporizer heated at 280 °C at a rate of 12.4g/hr together with nitrogen (14.4 NL/hr) and oxygen (0.95 NL/hr), so that glycerin was gasified. The resulting reactant gas including glycerin was passed through the CsPMo catalytic layer and then PW/$TiO_2$ catalytic layer. These catalytic layers were heated at 280 °C in an electric oven. The feed stream has a composition of glycerin: oxygen: nitrogen: water = 6.2 : 3.5 : 58.5 : 31.5. GHSV in first and second catalytic layer was 2500 $hr^{-1}$ and 10000 $hr^{-1}$ respectively. The reaction pressure was 1.7 bars at the gage pressure. The products were collected in a cooling condenser at an outlet of the reactor to determine a composition of gas.

Condensate and product gases were analyzed by gas chromatograph ( (HP6890 Agilent, FFAP column, FID detector, CP4900 Varian, Silicaplot and Molecular Sieve 5 A, TCD detectors). Absolute contents of products were calculated by using correcting factors in the products obtained by gas chromatograph.

Conversion of material, selectivity of objective substance and yield were calculated by following equation:

Conversion (%) of material = (a mole number of material reacted / a mole number of material fed) * 100

Selectivity of objective product (%) = (a mole number of objective product obtained / a mole number of material reacted) * 100

Yield of objective product (%) = (a mole number of objective product obtained / a mole number of material fed) * 100

Molar ratio of propionaldehyde to acrolein = (a mole number of propionaldehyde produced / a mole number of acrolein produced)

Result is shown in [Table 4].

Example 22 (Comparative Example)

Reaction was evaluated by repeating Example 21, but catalyst was changed to $PW/TiO_2$ (9.6ml). Result is shown in [Table 4].

Table 4

| Example | 21 | 22 (comparative) |
|---|---|---|
| Glycerol conversion (%) | >99 | >99 |
| Acrolein yield (%) | 49 | 76 |
| Acrylic acid yield (%) | 25 | 0.3 |
| Popionaldehyde yield (%) | 0.02 | 0.7 |
| Propionic acid yield (%) | 0.2 | 0.13 |
| Acetic acid yield (%) | 8.6 | 0.4 |
| Acetaldehyde yield (%) | 2.3 | 1.6 |
| Hydroxypropanone yield (%) | 0 | 0.2 |
| Propionaldehyde/acrolein molar ratio (%) | 0.04 | 0.92 |

Example 23:

Acrylic acid production from glycerol with a step of selective elimination of propanal

A simulation using the ASPEN PLUS® software was used to illustrate the method according to the invention. In this example, %wt means % by weight and ppmwt means part per million by weight. We mentioned only the components at a concentration above 1%wt.

A gas stream at 320°C under 2.8 bar (82.5 t/h, 20.8%wt glycerol, 51.7%wt water, 21.8%wt carbon dioxide, 5.3%wt oxygen) is sent to a multitube fixed bed reactor containing a heterogeneous dehydration catalyst coupled with a molten salt bath. A gas stream leaves this reactor at 320°C under 1.8 bar (82.5 t/h, 59.8%wt water, 4.2%wt oxygen, 10.2%wt acrolein, 22.4%wt $CO_2$). This gas stream contains 650 ppmwt of propanal. This stream is cooled to 102 °C in a series of heat exchangers. A liquid phase (26.7 t/h, 97%wt water) is removed and the gas phase is sent to the lower part of condensation column, that comprises a condenser at its top to generate a liquid reflux in the column. Another gaseous flow is injected at the bottom of the column (35.9 t/h, 123°C, 76.3%wt $CO_2$ 16.5%wt water, 3.0%wt CO, 1.9%wt $O_2$), The gas phase that goes out the condenser (69.5 t/h, 74°C, 1.7 bar, 65.9%wt $CO_2$, 12.1%wt acrolein, 10.8%wt water, 6.0%wt $O_2$, 2.4%wt CO, 1.3%wt acetaldehyde, 770 ppmwt propanal) is heated to 240°C in a heat exchanger.

This stream is sent to the top of a multitubular fixed bed reactor that comprises 2 sections. Each section is coupled with a specific molten salt bath. The upper section contains a catalyst for selective elimination of propanal and the salt bath of this section is kept at 300°C. The lower section contains a catalyst for the oxidation of acrolein to acrylic acid and the salt bath of this section is heated to 260°C. After the first section, the gas flow contains 66.6%wt $CO_2$, 11.8%wt acrolein, 11.0%wt water, 5.4%wt $O_2$, 2.4%wt CO, 1.3%wt acetaldehyde, 120 ppmwt propanal. At the outlet of the reactor, the gas stream contains 67.2%wt $CO_2$, 14.4%wt acrylic acid, 11.2%wt water, 1.1%wt $O_2$, 2.6%wt CO, 1.1%wt acetic acid, and 70 ppm propionic acid.

This stream is cooled to 160 °C and injected in an absorption column. At the top of this column, water (6.5 t/h) is injected. The liquid that is recovered at the bottom of the column is send to a second column that is operated under vacuum. A stream of acrylic acid is recovered at the bottom of this column (15.5 t/h, 63.6%wt acrylic acid, 27.7 wt% water, 4.9%wt acetic acid, 3.0wt% formic acid and 300 ppmwt propionic acid).

Example 24 (comparative):

Acrylic acid production from glycerol without selective elimination of propanal

The same simulation as Example 23 was reproduced with a second multitubular fixed bed reactor that comprises only one section. This section contains a catalyst for the oxidation of acrolein to acrylic acid and the salt bath of this section is heated to 260°C. The stream of acrylic acid that is recovered at the bottom of the last column contains 63.8%wt acrylic acid, 27.7 wt% water, 4.9%wt acetic acid, 3.0wt% formic acid and 2000 ppmwt propionic acid.

Example 25:

Methylmercaptopropionaldehyde production from glycerol with selective elimination of propanal

The same experiment as Example 21 is reproduced. The hot acrolein gases out of the reactor are quenched in cold water in order to get an aqueous phase containing around 3%wt acrolein. This aqueous phase is further distilled. Acrolein containing 96%wt acrolein, 0.4%wt acetaldehyde, 3% wt water and 0.04%wt propanal is recovered. A reaction mixture consisting of 25 g of methylmercapto- propionaldehyde and 0.85 g of a mixture of 48%wt of pyridine in acetic acid is introduced in a 250 ml reactor, At 60°C, 89.4 g of the obtained acrolein and 74.9 g of 99.5%wt methylmercaptan are simultaneously introduced in this reaction mixture over a 30 minute period and reaction is continued for 10 minutes then the mixture is cooled. Methylmercaptopropionaldehyde is obtained and can be used without further purification or with a purification by distillation under reduced pressure for methionine or 2-hydroxy-4-(methylthio)butyric acid synthesis.

Followings observations are observed from Examples and Comparative Examples.

(1) Improved performance in reduction of propionaldehyde with such higher conversion of propionaldehyde than 90 % by using the catalyst according to this invention, containing Mo as an essential component, and at least one element selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al, preferably from a group comprising P, Si, W, Ti, Cr, Mn, Fe, Co, Ni, Zn, Sn, Bi, Sb, Ce, Mg, Cs and K, in particularly, by using the catalyst comprising Mo-containing heteropolyacids such as CsPMo and MoVPCu or their salts and Mo-containing composite metal oxides such as FeMo and MoBi.

(2) In a case of $TiO_2$ alone, which is outside of this invention, the propionaldehyde conversion is higher than the conversion of acrolein.

(3) The content of propionaldehyde can be reduced such greatly as is shown in the molar ratio of propionaldehyde to acrolein of 0.04 for the catalyst of this invention, comparing to 0.92 for those that do not use the catalyst of this invention, by using the catalyst according to this invention, containing Mo as an essential component, and at least one element selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al, preferably from a group comprising P, Si, W, Ti, Cr, Mn, Fe, Co, Ni, Zn, Sn, Bi, Sb, Ce, Mg, Cs and K, in particularly, by using the catalyst comprising Mo-containing heteropolyacids of CsPMo.

## Claims

1. Catalyst for use in selective reduction of propionaldehyde in acrolein containing the propionaldehyde, **characterized in that** the catalyst contains Mo as an indispensable component, and at least one element selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al.

2. The catalyst of claim 1, wherein the catalyst contains Mo as an indispensable component, and at least one element selected from a group comprising P, Si, W, Ti, Cr, Mn, Fe, Co, Ni, Zn, Ga, Sn, Bi, Sb, Ce, Mg, Cs and K.

3. The catalyst of claim 1 or 2, wherein the catalyst comprises a compound represented by the general formula (1):

$$A_aX_bY_cZ_dO_e \qquad (1)$$

in which,

A is at least one cation selected from elements belonging to the Group I to Group 16 in the Periodic Table,

X is P or Si,
Y is Mo,
Z is at least one element selected from a group comprising W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al,
a, b, c and d in the formula each satisfy respective range of $0 \leq a < 9$, $0 \leq b \leq 1{,}0 < c \leq 20$ and $0 < d \leq 20$, and d is a value determined by oxidation numbers of other elements.

**4.** The catalyst of claim 3, wherein Z is at least one element selected from a group comprising W, Ti, Cr, Mn, Fe, Co, Ni, Tl, Zn, Sn, Bi, Sb, Mg, K, Rb and Cs.

**5.** The catalyst of claim 4, wherein the catalyst comprises a compound represented by the general formula (2):

$$Mo_{12}X_bO_c \qquad (2)$$

in which,

X is at least one element selected from a group comprising W, Ti, Cr, Mn, Fe, Co, Ni, Tl, Zn, Sn, Bi, Sb, Mg, K, Rb and Cs,
b is a number of atom of X and satisfies a range of $0 < b < 30$, and
c is a value determined by oxidation numbers of other elements.

**6.** The catalyst of any one of claims 1 to 5, wherein the catalyst consists mainly of heteropolyacid containing Mo as an indispensable component.

**7.** The catalyst of any one of claims 1 to 5, wherein the catalyst consists mainly of a compound in which protons in a heteropolyacid containing Mo as an indispensable component are exchanged with at least one cation of elements belonging to the Group I to Group 16 in the Periodic Table.

**8.** The catalyst of claim 7, wherein said cation is cation of alkali metals.

**9.** The catalyst of claim 8, wherein said alkali metal is Cs.

**10.** The catalyst of any one of claims 1 to 9, wherein said compound exchanged with said cation is added further with at least one salt of elements belonging to the Group I to Group 16 in the Periodic Table.

**11.** Supported catalyst comprising said catalyst defined in any one of claims 1 to 10, supported on a carrier.

**12.** Supported catalyst of claim 11, wherein said carrier is at least one metal oxide selected from a group comprising titania, silica, zirconia, niobia, magnesia, ceria and alumina.

**13.** A process for preparing a catalyst for use in selective reduction of propionaldehyde in acrolein containing the propionaldehyde, by the steps of adding at least one salt or oxide of elements selected from a group comprising P, Si, W, Ti, Zr, V, Nb, Ta, Cr, Mn, Fe, Co, Ni, Cu, Zn, Ga, In, Tl, Sn, Ag, As, Ge, B, Bi, La, Ba, Sb, Te, Ce, Pb, Mg, K, Rb, Cs and Al into a solution containing a salt or oxide of Mo, and firing or calcinating the resulting solid product.

**14.** The process of claim 13, wherein at least one salt or oxide of elements selected from a group comprising P, Si, W, Ti, Cr, Mn, Fe, Co, Ni, Zn, Ga, Sn, Bi, Sb, Ce, Mg, Cs and K into a solution containing a salt or oxide of Mo.

**15.** The process of claim 13 or 14, wherein the firing or calcination is carried out in air, in inert gas, in a mixture of oxygen and inert gas or in an atmosphere of hydrogen and inert gas.

**16.** The process of any one of claims 13 to 15, wherein the firing or calcination is carried out at a temperature of from 150°C to 900°C for 0.5 to 10 hours.

**17.** Use of the catalyst define in any one of claims 1 to 10 or the carried catalyst define in claim 11 and 12, in selective reduction of the propionaldehyde in acrolein containing the propionaldehyde.

**18.** Use of the catalyst define in any one of claims 1 to 10 and/or the carried catalyst define in claim 11 and 12, in selective reduction of the propionaldehyde in acrolein containing the propionaldehyde in which a molar ratio of propionaldehyde to acrolein is from 1/1000 to 1/5.

**19.** Use of the catalyst define in any one of claims 1 to 10 and/or the carried catalyst define in claim 11 and 12, in selective reduction of the propionaldehyde in acrolein which is obtained by catalytic dehydration of glycerin.

**20.** The catalyst define in any one of claims 1 to 10 and/or the carried catalyst define in claim 11 and 12, used in selective reduction of the propionaldehyde in acrolein containing the propionaldehyde, which is obtained by oxidation of propylene.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5387720 A
- FR 695931
- WO 2006087084 A
- WO 2009044081 A
- WO 2009128555 A
- WO 2010046227 A
- WO 2010074177 A
- US 4381411 A
- JP 54046705 A
- WO 2009127889 A
- JP 10218831 A
- EP 2039674 A
- JP 3793317 B
- WO 2006087083 A
- WO 200944081 A

### Non-patent literature cited in the description


- **HARGIS et al.** *I&EC product research and development,* March 1966, vol. 5 (1), 72-75
- *Kinetics and Catalysis,* 2003, vol. 44 (2), 198-201
- **JI HU et al.** reports oxidative dehydrogenation reaction of isobutyl aldehyde to methacrolein in PMo catalyst. *Journal of Catalysis,* 2000, vol. 195, 360-375
- **CICMANEC et al.** repeated the same reaction by using CsPMo catalyst. *React. Kinet. Catal. Lett.,* 2004, vol. 81 (2), 383-391
- **HARGIS et al.** *I & EC product research and development,* March 1966, vol. 5 (1), 72-75
- *Journal of Catalysis,* 2000, vol. 195, 360-375
- *React. Kinet. Catal. Lett.,* 2004, vol. 81 (2), 383-391